# EUROPEAN PATENT APPLICATION

(11) **EP 2 775 304 A1**
(43) Date of publication of application: **10.09.2014**
(21) Application number: 13158225.6
(22) Date of filing: 07.03.2013
(51) Int. Cl.: G01N 33/53

(54) **Methods for detecting inflammatory disorder**

(71) Applicant: Universitätsspital Basel, 4031 Basel (CH); Kantonsspital Aarau AG, 5001 Aarau (CH)
(72) Inventor: Hahn, Sinuhe, 4410 Liestal (CH); Hasler, Paul, 5000 Aarau (CH); Giaglis, Stavros, 4057 Basel (CH); Chowdury, Chanchal Sur, 4056 Basel (CH)
(74) Representative: Kanopka, Martin

(57) **Abstract**

The present invention relates to methods and compositions for detecting inflammatory disorders and more particularly to methods and composition for detecting inflammatory disorders by detecting cell-free nucleosomes in a serum or plasma sample isolated from a subject.

## Description

### FIELD OF THE INVENTION

The present invention relates to methods and compositions for detecting inflammatory disorders and more particularly to methods and compositions for detecting inflammatory disorders by detecting cell-free nucleosomes in serum or plasma samples isolated from a subject. In addition, the present invention relates to methods for monitoring the efficacy of a therapeutic agent used for treating an inflammatory disorder.

### BACKGROUND ART

Pain, stiffness and swelling of the joints are the clinical correlates of synovial inflammation in rheumatoid arthritis, which is initiated by the extravasation of polymorphonuclear granulocytes into the synovium, followed by monocytes/macrophages and subsequently by T and B cells *(*R. N. Maini, M. Feldmann, Cytokine therapy in rheumatoid arthritis, Lancet 348, 824 (Sep 21, 1996*).*

The macrophages, T cells, and polymorphonuclear granulocytes combine with local synoviocyte-like fibroblasts to form the pannus, an invasive tissue that erodes the cartilage and adjacent bone of the joint. Unchecked erosion and cyst formation ultimately lead to the loss of integrity and function of the joints and consequently, invalidity *(*N. Wegner et al., Autoimmunity to specific citrullinated proteins gives the first clues to the aetiology of rheumatoid arthritis. Immunol Rev 233, 34 (Jan, 2010*)).*

A novel feature of polymorphonuclear granulocyte biology is their ability to generate neutrophil extracellular traps *(*V. Brinkmann et al., Neutrophil extracellular traps kill bacteria. Science 303, 1532 (Mar 5, 2004*)),* via a distinct process of cell death termed NETosis *(*T. A. Fuchs et al., Novel cell death program leads to neutrophil extracellular traps. J Cell Biol 176, 231 (Jan 15, 2007*)).* Neutrophil extracellular traps consist of chromosomal extruded DNA decorated with granular components which include antimicrobial peptides and proteases. The molecular pathways leading to NETosis involve reactive oxygen species generation (ROS), calcium mobilization and chromatin modification, in particular the citrullination of histone 3 (H3) by peptidyl arginine deiminase 4 (PAD4) (See for example: T. A. Fuchs et al., Novel cell death program leads to neutrophil extracellular traps. J Cell Biol 176, 231 (Jan 15, 2007*)).*

Recently, neutrophil extracellular traps have become implicated in the aetiology auto-inflammatory conditions such as preeclampsia, Felty's syndrome, systemic lupus erythematosus (SLE) and multiple sclerosis (MS) (See for example, A. K. Gupta, P. Hasler, W. Holzgreve, S. Gebhardt, S. Hahn, Induction of neutrophil extracellular DNA lattices by placental microparticles and IL-8 and their presence in preeclampsia. Hum Immunol 66, 1146 (Nov, 2005*);* R. Lande et al., Neutrophils activate plasmacytoid dendritic cells by releasing self-DNA-peptide complexes in systemic lupus erythematosus. Science translational medicine 3, 73ra19 (Mar 9, 2011*);* N. Dwivedi et al., Felty's syndrome autoantibodies bind to deiminated histones and neutrophil extracellular chromatin traps, Arthritis and Rheumatism 64, 982 (April 2012*)).*

Previously, a significant increase in concentrations of cell-free DNA in the sera of rheumatoid arthritis patients compared with healthy controls has been detected *(*X. Y. Zhong et al., Increased concentrations of antibody-bound circulatory cell-free DNA in rheumatoid arthritis, Clin Chem 53, 1609 (Sep, 2007*)).* A link between circulating cell-free DNA and neutrophil extracellular traps has previously been made in preeclampsia, where the presence of neutrophil extracellular traps in the sinusoids of the placenta correlated with elevated cell-free DNA concentrations (See for example: A. Gupta, P. Hasler, S. Gebhardt, W. Holzgreve, S. Hahn, Occurrence of neutrophil extracellular DNA traps (Neutrophil extracellular traps) in preeclampsia: a link with elevated levels of cell-free DNA? Ann N Y Acad Sci 1075, 118 (Sep, 2006*)).*

### DISCLOSURE OF THE INVENTION

### THE PROBLEMS AND THEIR SOLUTION

Inflammatory disorders, such as rheumatoid arthritis are difficult to detect prior to the manifestation of clinical symptoms such as pain, stiffness and swelling of joints. Unfortunately, at this stage, considerable destruction of the joint by an autoimmune response may have taken place. Recent data has indicated that the early detection of rheumatoid arthritis and rapid initiation of treatment with effective biologics-based therapies led to significantly better patient outcome, and can prevent widespread joint damage *(*H. Zeidler, The need to better classify and diagnose early and very early rheumatoid arthritis. The Journal of rheumatology 39 (2012) 212-7*.)*

Current tests such as those for rheumatoid factor or antibodies to citrullinated peptides (hereinafter antibodies to citrullinated peptides will be referred to as "ACPA") have limited use, as they have low specificity for rheumatoid arthritis and/or are restricted to a sub-group of rheumatoid arthritis patients (i.e. only those which are ACPA positive). Although the presence of ACPA has high specificity for the development of rheumatoid arthritis and better predictive value than the occurrence of rheumatoid factor, they are only present in approximately two-thirds of rheumatoid arthritis patients *(*N. Wegner et al., Autoimmunity to specific citrullinated proteins gives the first clues to the aetiology of rheumatoid arthritis. Immunol Rev 233, 34 (Jan, 2010*);* A. Willemze, L. A. Trouw, R. E. Toes, T. W. Huizinga, The influence of ACPA status and characteristics on the course of RA. Nat Rev Rheumatol 8, 144 (Mar, 2012*)).* In addition, ACPA appear to be strongly associated with the "shared epitope" on HLA-DRB1, and may be subject to ethnic differences *(*N. Wegner et al., Autoimmunity to specific citrullinated proteins gives the first clues to the etiology of rheumatoid arthritis. Immunol Rev 233, 34 (Jan, 2010*);* A. Willemze, L. A. Trouw, R. E. Toes, T. W. Huizinga, The influence of ACPA status and characteristics on the course of RA. Nat Rev Rheumatol 8, 144 (Mar, 2012*)).*

In addition, presently the inflammatory disorder activity such rheumatoid arthritis disease activity is monitored or assessed through clinical observations such as pain, stiffness, functional abilities, and swollen joints, i.e. through external signs and symptoms of a subject. For example, rheumatoid arthritis disease activity is monitored through DAS28 or DAS44, disease activity scores incorporating an assessment of 28 or 44 joints for swelling and tenderness together with a laboratory parameter indicating inflammation, in this case the erythrocyte sedimentation rate or the C-reactive protein. Other measures for disease activity can be validated patient questionnaires alone, such as the rheumatoid arthritis disease activity score (RADAI), or combined with other assessment tools. However, these clinical and laboratory monitoring or assessment methods are non-specific for an inflammatory disorder, in particular, they are non-specific for rheumatoid arthritis. Furthermore, they are invasive, time consuming, they cannot be used to predict the course of the disorder, nor can they be used to monitor the efficacy of therapeutic agent. Therefore, it is desirable to find an alternative biomarker to detect inflammatory disorders, in particular, rheumatoid arthritis at an early stage, optionally, prior to appearance of clinical symptoms.

In addition, it is desirable to find an alternative biomarker to detect inflammatory disorders such as preeclampsia, where manifestation of clinical symptoms can occur within a matter of hours and require expeditious intervention to prevent serious harm to a patient. Furthermore, it is desirable to find an alternative biomarker, methods, and compositions with higher specificity for inflammatory disorders, in particular, for rheumatoid arthritis than the current tests and to find an alternative biomarker, methods, and compositions that are not restricted to a particular patient sub-group.

It is also desirable to find an alternative biomarker to monitor or predict the efficacy of a therapeutic agent used for treating inflammatory disorders, in particular, rheumatoid arthritis. Accordingly, it is desirable to find an alternative biomarker with high specificity to tailor a suitable treatment for an inflammatory disorder and to develop anti-inflammatory drugs.

It is also desirable to find an alternative method of monitoring an inflammatory disorder activity, in particular, rheumatoid arthritis disease activity in order to optimize the treatment of disease, to predict the future course of disease, and to assess efficacy of a therapeutic agent for treatment of disease.

Ultimately, it is desirable to improve diagnosis and prognosis of inflammatory disorders, in particular, rheumatoid arthritis.

The present invention provides such biomarker to solve the problems mentioned above, and methods and compositions to detect inflammatory disorders, in particular, rheumatoid arthritis. Other features and advantages of the invention will be apparent from the following description and from the claims.

### SUMMARY OF THE INVENTION

The summary is not intended to limit the scope of the present invention. Additional embodiments will be apparent from the detailed description, drawings and from the claims. The present invention provides a method for detecting an inflammatory disorder in a subject comprising detecting cell-free nucleosomes in a serum or plasma sample isolated from said subject, wherein the presence of the cell-free nucleosomes is indicative of the presence of said inflammatory disorder.

In further embodiment, the present invention provides a method for predicting or monitoring the efficacy of a therapeutic agent for use in treating an inflammatory disorder comprising measuring the level of cell-free nucleosomes in a serum or plasma sample isolated from a subject pre and post treatment, wherein a change in the level of said cell-free nucleosome indicates efficacy of said agent.

In further embodiment, the present invention provides a method for the selection of a therapeutic agent for use in treating of an inflammatory disorder comprising measuring the level of cell-free nucleosomes in a serum or plasma sample isolated from a subject pre and post treatment, wherein a suitable therapeutic agent is selected based on the level of the cell-free nucleosome in the sample.

The therapeutic agent, optionally, comprises a pharmaceutical composition, a signaling molecule, cells, manipulated cells, or combinations thereof. In another embodiment, the therapeutic agent is used for extracorporeal treatment of the subject.

In further embodiment, the present invention provides use of cell-free nucleosomes in detecting, diagnosing, prognosing an inflammatory disorder, in monitoring and/or predicting the efficacy of a therapeutic agent on an inflammatory disorder, in selecting a therapeutic agent for use in treatment of an inflammatory disorder, in detecting NETosis, in monitoring an inflammatory disorder activity, or in detecting the effect of an environmental stimulus on a subject.

In further embodiment, the present invention provides a method for monitoring an inflammatory disorder activity. Said method comprises measuring the level of cell-free nucleosomes in a serum or plasma sample isolated from a subject, correlating the measured level of cell-free nucleosomes with the activity of the inflammatory disorder, wherein the measured level of cell-free nucleosomes in the sample compared to a pre-determined value indicates the extent of activity.

In further embodiment, the present invention provides a method for detecting NETosis in a subject, said method comprises detecting cell-free nucleosomes in a serum or plasma sample isolated from said subject, wherein the presence of the cell-free nucleosomes is indicative of NETosis.

In further embodiment, the present invention provides a method for detecting the effect of an environmental stimulus on a subject. The method comprises measuring the level of cell free nucleosomes in a serum or plasma sample, correlating the measured level of cell-free nucleosomes with the effect of the stimulus on the subject, wherein the measured level of cell-free nucleosomes in the sample compared to a pre-determined value indicates the extent of the stimulus effect on the subject.

In further embodiment, the present invention provides a method for detecting an inflammatory disorder in a subject comprising detecting cell-free nucleosomes in a serum or plasma sample isolated from said subject, wherein the presence of the cell-free nucleosomes is indicative of the presence of said inflammatory disorder and wherein detecting the cell-free nucleosomes in the serum or plasma sample isolated from said subject comprises contacting the serum or plasma sample with one or more reagents for detection of the cell-free nucleosomes and detecting specific binding of the one or more reagents with the cell-free nucleosomes.

In further embodiment, the one or more reagents according to the preceding embodiment, binds specifically with a modified histone protein and/or a modified DNA of the cell-free nucleosome and wherein specific binding of the one or more reagents to the modified histone protein and/or a modified DNA of the cell-free nucleosome is indicative of the presence of said inflammatory disorder.

In further embodiment, the present invention provides a method for detecting an inflammatory disorder in a subject comprising detecting cell-free nucleosomes in a serum or plasma sample isolated from said subject, wherein the presence of the cell-free nucleosomes is indicative of the presence of said inflammatory disorder and wherein detecting the cell-free nucleosomes in the serum or plasma sample isolated from said subject comprises contacting the serum or plasma sample with a first reagent and a second reagent, wherein one of said first or said second reagent binds specifically with a DNA or a modified DNA of the cell-free nucleosome and the other of said first or second reagent binds specifically with a histone protein or a modified histone protein of the cell-free nucleosome.

In another embodiment, detecting the cell-free nucleosomes according to any of the preceding embodiments further comprises immobilizing the cell-free nucleosomes or the modified histones of the cell-free nucleosomes on a solid suspended in a liquid.

In another embodiment, said one or more reagents or said first and second reagent according to any of the preceding embodiments is detectably labeled.

In another embodiment, said one or more reagents or said first and second reagent according to any of the preceding embodiments is an antibody, an aptamer, series of antibodies or aptamers, or combinations thereof.

In another embodiment, the present invention provides a composition comprising one or more reagents which binds specifically with cell-free nucleosomes in a serum or plasma sample isolated from a subject for use in detecting, monitoring, diagnosing, and/or making a prognosis of an inflammatory disorder.

In another embodiment, the one or more reagents of the composition according to the preceding embodiment, binds specifically with a modified histone protein and/or modified DNA of the cell-free nucleosome.

In another embodiment, the present invention provides a composition comprising one or more reagents which binds specifically with cell-free nucleosomes in a serum or plasma sample isolated from a subject for use in detecting, monitoring, diagnosing, and/or making a prognosis of an inflammatory disorder, wherein the reagents comprises a first reagent and a second reagent, wherein one of said first or said second reagent binds specifically with a DNA or a modified DNA of the cell-free nucleosome and the other of said first or second reagent binds specifically with a histone protein or a modified histone protein of the cell-free nucleosome.

In another embodiment, said one or more reagents or said first and second reagent of the composition according to any of the preceding embodiments is detectably labeled.

In another embodiment, said one or more reagents or said first and second reagent of the composition according to any of the preceding embodiments, is an antibody, an aptamer, series of antibodies or aptamers, or combinations thereof.

In another embodiment, the present invention provides a method for detecting an inflammatory disorder in a subject comprising administering to a serum or plasma sample isolated from a subject a composition according to any of the preceding embodiments and detecting the binding of said one or more reagents or said first and second reagent with the cell-free nucleosomes or with the modified DNA or modified histone protein of the cell-free nucleosomes, wherein specific binding of said one or more reagents or said first and second reagent is indicative of the presence of said inflammatory disorder. Preferably, said one or more reagents or said first and second reagent according to this embodiment is an antibody, an aptamer, series of antibodies or aptamers, or combinations thereof.

In another embodiment, the modified histone protein of the cell-free nucleosomes according to the methods and the compositions of any of the preceding embodiments has a modification selected from histone deimination, histone methylation, histone phosphorylation or histone acetylation. Optionally, the histone deimination is histone 3 (H3), histone 2A (H2A) and histone 4 (H4) deimination.

In another embodiment, the modified DNA of the cell-free nucleosomes according to the methods and the compositions of any of the preceding embodiments has a modification selected from DNA acetylation or DNA demethylation.

In another embodiment, the inflammatory disorder according to the present invention is rheumatoid arthritis.

In another embodiment, the inflammatory disorder according to the present invention is preeclampsia.

In further embodiment, the inflammatory disorder according to the present invention is systemic lupus erythematosus, Felty's syndrome, anti-neutrophil cytoplasmic antibody associated vasculitis including granulomatosis with polyangiitis, localized infection, infection of body orifices and bloodstream infection, sepsis, or multiple sclerosis.

In another embodiment, the subject according to the methods and the compositions of any of the preceding embodiments is a living multi-cellular vertebrate organism. Optionally, the subject is a human and non-human mammal. Non-human mammals are, optionally, canine, feline or equine.

Additional embodiments provide use of the methods and compositions according to any of the preceding embodiments in the diagnosis, prognosis, monitoring of an inflammatory disorder and provide kits comprising the compositions according to any embodiment of the present invention.

### DRAWINGS

Those of skill in the art will understand that the drawings, described below, are for illustrative purposes only. The drawings are not intended to limit the scope of the present teachings in any way.
Figure 1. Illustrates neutrophil, peripheral blood leukocyte counts and age distribution in rheumatoid arthritis (RA) cases and control cohorts.
Figure 1A. Illustrates neutrophil levels in cases with RA (n = 32) and matched controls - healthy blood donors (n = 41).
Figure 1B. Illustrates peripheral blood mononuclear cell count in RA cases and healthy control donors.
Figure 1C. Illustrates age distribution of RA cases and matched healthy blood donors.
Figure 2. Illustrates serum from patients with RA exhibits elevated levels of the main component of NETs.
Figure 2A. Illustrates cell-free DNA levels in plasma and serum from controls - healthy donors (n=41) and patients with RA (n=32) determined by real-time PCR.
Figure 2B. Illustrates cell-free nucleosome levels in plasma and serum from controls - healthy donors and patients with RA, determined by photometric ELISA-based quantitative detection of histone-associated DNA fragments in plasma and serum from controls - healthy donors and patients with rheumatoid arthritis.
Figure 2C. Illustrates determination of neutrophil elastase (NE) protein concentration in plasma and serum from healthy donors and patients with rheumatoid arthritis as assessed by sandwich ELISA.
Figure 2D. Illustrates myeloperoxidase concentrations in plasma and serum from healthy donors and patients with RA determined by sandwich ELISA.
Figure2E. Illustrates enzyme activity of NET-associated myeloperoxidase quantified utilizing a modified capture ELISA.
Figure 3. Illustrates ROC (receiver operator curve) analysis of cell free neutrophil derived analytes in serum and plasma samples from RA cases vs. healthy controls.
Figure 3A. Illustrates the ROC analysis of cell-free nucleosomes in serum (dark line) and plasma (light line).
Figure 3B. Illustrates the same analysis for cell-free DNA.
Figure 3C. Illustrates the ROC analysis for myeloperoxidase (MPO).
Figure 3D. Illustrates the ROC analysis for neutrophil elastase (NE).
Figure 4. Illustrates cell-free nucleosome levels prior and post immune modulating drug infusion.
Figure 5. This figure shows scanning electron micrograph of spontaneous NETosis observed *in-vitro* by polymorphonuclear granulocytes (PMN) isolated from healthy control donor or patient with RA.
Figure 6. Shows that RA-derived PMN undergo NETosis *in vitro* significantly more rapidly than control PMN.MPO protein expression, monitored through western blots, was significantly elevated in PMNs from patients with RA compared to control blood donors.
Figure 6A. Illustrates PMN from healthy controls and patients with rheumatoid arthritis which were incubated for a three-point time course in-vitro tissue culture to observe and analyze spontaneous NET formation via immunohistochemistry for NE and DAPI. Magnification: 20x; scale bar: 50 nm. After 3 hours of incubation NET formation was more pronounced in PMN from RA patients in comparison to control healthy neutrophils.
Figure 6B. Illustrates PMN culture supernatants from all time points were collected and concentration of cell-free nucleosomes was determined through capture ELISA.
Supernatants from RA-derived cultured PMN were found to contain significantly higher levels of cell-free nucleosomes compared to the ones corresponding to healthy donors PMN after 3 hours of incubation. *P = 0.05, ***P = 0.001, one-way ANOVA with Dunn's post test correction.
Figure 6C. Illustrates *in vitro* release of Neutrophil extracellular traps measured via enzyme activity of NET-associated myeloperoxidase through a modified capture ELISA approach confirmed the significantly higher NET formation capacity of the RA-derived PMN. *P = 0.05, **P = 0.01, ****P = 0.0001, one-way ANOVA with Dunn's post test correction.
Figure 6D. Illustrates three distinct PMN nuclear morphologies can be observed after immunohistochemical staining of neutrophils for NE and DAPI.
Figure 6E. Illustrates, according to that objective PMN nuclear morphology classification, the RA-derived PMN exert an excessive spontaneous change in nuclear decondensation during the 3 hour *in vitro* time course. Interestingly, the delobulated/diffused nuclear phenotype was significantly more pronounced in RA PMNs even from the 0h time point. *P = 0.05, ***P = 0.001, one-way ANOVA with Dunn's post test correction.
Figure 6F. Illustrates baseline ROS levels in resting PMN.
Figure 6G. Illustrates NE mRNA expression and (H.) MPO mRNA expression measured by real-time PCR, as well as Figure 21. MPO protein expression, monitored through western blots, was significantly elevated in PMNs from patients with RA compared to control blood donors. *P = 0.05, **P = 0.01, ***P = 0.001, ****P = 0.0001, Mann-Whitney t test with Welch's correction. Ctrl: control; RA: rheumatoid arthritis; MPO: myeloperoxidase; NE: neutrophil elastase; DAPI: 4',6-diamidino-2-phenylindole; n.s.: statistically not significant.
Figure 7. Examination of PAD4 expression in RA-derived PMN
Figure 7A. Illustrates quantification of PAD4 protein expression after western blot analysis (left panel) in total PMN cell extracts did not show any significant differences in the expression levels of PAD4 between the healthy controls and the RA patients, confirmed similarly in PAD4 mRNA expression levels according to Figure 7B.
Figure 7C. Illustrates after isolating the nuclear and cytoplasmic protein extracts, PAD4 protein levels in the RA PMN nuclei were significantly elevated compared to the healthy controls (lower right panel), whereas in the cytoplasmic fraction an opposite tendency was observed (upper right panel). *P = 0.05, ****P = 0.0001, Mann-Whitney t test with Welch's correction; n.s.: statistically not significant.
Figure 7D. Western blot analysis of citrullinated histone H3 (citH3), the natively occurring substrate of PAD4, levels indicates that these are present in greater quantities in PMN from RA cases than from healthy controls. ****P = 0.0001, Mann-Whitney t test with Welch's correction;
Figure 7E. Illustrates double immunohistochemical staining of PAD4 revealed localization of PAD4 protein on the neutrophil extracellular traps, which coincided with the extracellular DNA and the histones.
Figure 7F. Illustrates predominantly cytoplasmic PAD2 also co-localized on RA Neutrophil extracellular traps following the trajectory of extracellular DNA, stained with DAPI, in a rather diffuse fashion. Magnification: upper panel 20x, lower panel 40x.
Figure 7G. Illustrates co-localization of citrullinated histone H3 with the histones on the Neutrophil extracellular traps confirmed that PAD4 is active on the Neutrophil extracellular traps. Magnification: upper panel 20x, lower panel 63x.
Figure 8. Illustrates response of PMN from RA patients to secondary stimuli and influence of RA serum and synovial fluid on healthy PMN.
Figure 8A. Illustrates scanning electron micrographs of neutrophil extracellular traps induced by phorbol ester by control and RA-derived PMN. Note the excessive degree of NETosis undergone by RA-derived PMN.
Figure 8B. Illustrates the vast increase in the number of neutrophil extracellular traps induced by phorbol ester treatment by RA-derived PMN compared to controls, as assessed by fluorescent immunohistochemistry for MPO and DAPI.
Figure 8C. Illustrates the changes in PMN nuclear morphology following phorbol ester treatment, whereby the majority of RA-derived PMN change to a spread NETotic phenotype, with a concomitant decrease in the normal lobulated an the primed delobulated populations.
Figure 8D. Illustrates quantification of NETosis via the measurement of cell-free nucleosome released into the extracellular media following phorbol ester treatment, and its reduction by the PAD4 inhibitor, chloramidine.
Figure 8E. Illustrates increase nuclear localization of PAD4 triggered by phorbol ester treatment.
Figure 8F. Illustrates that RA derived serum (Se) and synovial fluid (SF) condition normal PMN to undergo enhanced NETosis.
Figure 9. RA-derived serum and synovial fluid activate normal PMN
Figure 9A. RA-derived serum (Se) and synovial fluid (SF) activate normal PMN as is evident by the increase in ROS production.
Figure 9B. Treatment with RA sera and SF lead trigger an increase in NETosis by normal PMN in-vitro as measured by the quantification of cell-free nucleosomes in the culture media. *P = 0.05, **P = 0.01, one-way ANOVA with Dunn's post-test correction. All data are representative of at least three independent experiments.
Figure 10. Illustrates a variety of histone modifications.

### DETAILED DESCRIPTION OF THE INVENTION

In one aspect of the invention, a method for detecting an inflammatory disorder is provided. In one embodiment, the inflammatory disorder according to the present invention and according to any of the disclosed embodiments is rheumatoid arthritis. Optionally, the inflammatory disorder is rheumatoid arthritis both positive and negative for anti-citrullinated peptide antibodies (ACPA). In the present disclosure, the terms "rheumatoid arthritis" and its abbreviation "RA" are used interchangeably.

In another embodiment, the inflammatory disorder according to the present invention and according to any of the disclosed embodiments is preeclampsia, systemic lupus erythematosus, Felty's syndrome, anti-neutrophil cytoplasmic antibody associated vasculitis including granulomatosis with polyangiitis, localized infection, infection of body orifices and bloodstream infection, sepsis, or multiple sclerosis.

The subject according to the present invention is a living multi-cellular vertebrate organism, a category that includes both human and non-human mammals. Optionally, the non-human mammals include equine, feline, or canine. The subject, optionally, includes cells or modified cell cultures obtained from said living multi-cellular vertebrate organism.

The method for detecting an inflammatory disorder according to the present invention comprises detecting cell-free nucleosomes in a serum or plasma sample isolated from said subject, wherein the presence of the cell-free nucleosomes is indicative of the presence of said inflammatory disorder.

The nucleosome is a unit consisting of approximately 200 bases pairs of chromosomal double strand DNA complexed with histones H1, H2A, H2B, H3 and H4. Nucleosomes form the basic unit of the structure of chromosomes in the nucleus of eukaryotic cells. During compromise of the integrity of the cell plasma and nuclear membranes, chromosomes may gain access to the extracellular space and lose connection to the cell of origin. Due to cleavage by DNAses, the DNA strand is severed at the intersection between nucleosomal units, releasing nucleosomes of many different units, thus becoming cell-free nucleosomes. In the present disclosure, the terms "cell-free nucleosome(s)", "cell-free DNA/histone complex" or "cell-free DNA/nucleosome complex" are used interchangeably. The detection of the cell-free nucleosomes in the serum or plasma sample isolated from said subject according to the methods of the present invention comprises contacting the serum or plasma sample with one or more reagents for detection of the cell-free nucleosomes and detecting specific binding of the one or more reagents with the cell-free nucleosomes. In one embodiment, the one or more reagents binds specifically with a histone or modified histone protein and with a DNA or modified DNA of the cell-free nucleosome and wherein specific binding of the one or more reagents to the histone or modified histone protein and to DNA or modified DNA of the cell-free nucleosome is indicative of the presence of said inflammatory disorder. Optionally, the one or more reagents binds specifically with a histone protein and modified DNA of the cell-free nucleosome. Optionally, the one or more reagents binds specifically with a modified histone protein and DNA of the cell-free nucleosome.

In another embodiment, the one or more reagents binds specifically with a modified histone protein and/or a modified DNA of the cell-free nucleosome and wherein specific binding of the one or more reagents to the modified histone protein and/or a modified DNA of the cell-free nucleosome is indicative of the presence of said inflammatory disorder.

In another embodiment, the detection of the cell-free nucleosome in a serum or plasma sample isolated from said subject according to the methods of present invention comprises contacting the serum or plasma sample with a first reagent and a second reagent, wherein one of said first or said second reagent binds specifically with a DNA or a modified DNA of the cell-free nucleosome and the other of said first or second reagent binds specifically with a histone protein or a modified histone protein of the cell-free nucleosome. Optionally, one of said first or said second reagent binds specifically with a DNA of the cell-free nucleosome and the other of said or second reagent binds specifically with a modified histone protein of the cell-free nucleosome. Optionally, one of said first or said second reagent binds specifically with a modified DNA of the cell-free nucleosome and the other of said or second reagent binds specifically with a histone protein of the cell-free nucleosome. Optionally, detecting the cell-free nucleosomes according to any of the preceding embodiments further comprises immobilizing or fixing the cell-free nucleosomes or the modified histones of the cell-free nucleosomes on a solid such as microplate module suspended in a liquid. The said immobilization can be performed by any method, on any solid, or in any liquid known in art. The one or more reagents or the first and second reagent of the preceding embodiments can be detectably labeled.

The one or more reagents or the first and second reagent of the present invention and according to any disclosed embodiment is, preferably, an antibody, an aptamer, series of antibodies or aptamers, or combinations thereof.

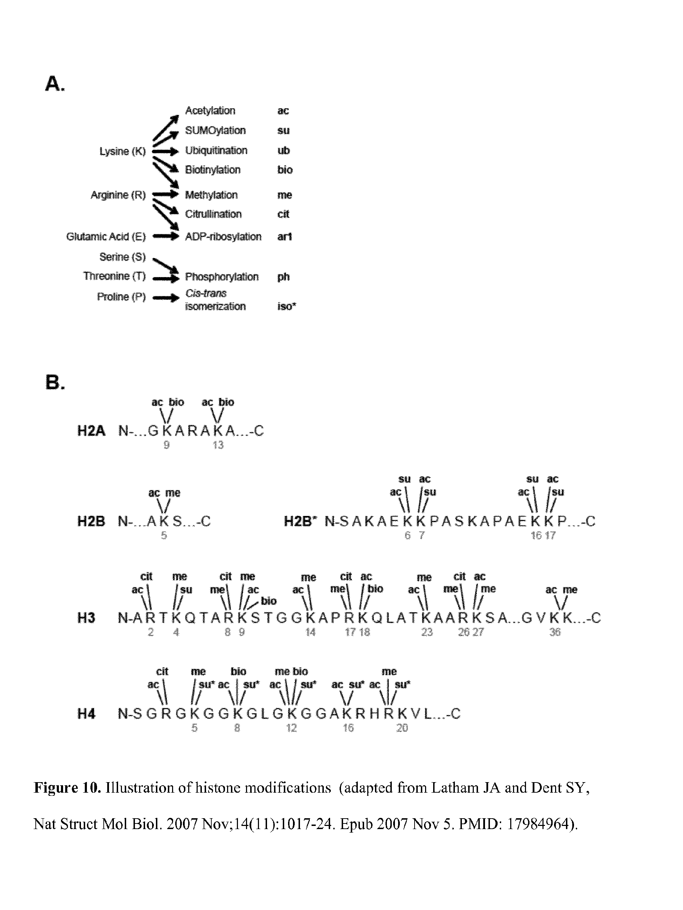

The modified histone protein of the cell-free nucleosome according to the present invention and any disclosed embodiment has, preferably, a modification selected from histone deimination, histone methylation, histone phosphorylation or histone acetylation. More preferably, the histone deimination is histone 3 (H3), histone H2A (H2A) and/or histone H4 (H4) deimination. Figure 10 illustrates, as an example, histone modifications (John A Latham & Sharon Y R Dent, Nature Structural & Molecular Biology 14, 1017 - 1024 (2007)).

In this description, the terms "citrullination" or "deimination" are used interchangeably. These terms denote to the post-translational modification of the amino acid_arginine in a protein into the amino acid citrulline. "Citrullination" or "deimination" involves removal of an amine group from arginine, converting it to citrulline. This reaction is performed by enzymes called peptidylarginine deiminases (PADs). Note that citrullination of proteins is distinct from the formation of the free amino acid citrulline as part of the urea cycle or as a byproduct of enzymes of the nitric oxide synthase family.

The modified DNA of the cell-free nucleosome according to the present invention and any disclosed embodiment, preferably, has a modification selected from DNA acetylation or DNA demethylation.

In further embodiment, the method according to any of the preceding embodiments further comprises diagnosing, monitoring, and making a prognosis of an inflammatory disorder, optionally, rheumatoid arthritis.

In additional embodiments the present invention provides use of the methods according to any of the disclosed embodiments in diagnosing, prognosing, and/or monitoring of an inflammatory disorder.

In another aspect of the invention a method for predicting or monitoring the efficacy of a therapeutic agent for use in treating an inflammatory disorder, optionally, rheumatoid arthritis is provided. Said method comprises measuring the level of cell-free nucleosomes in a serum or plasma sample isolated from a subject pre and post treatment, wherein a change in the level of said cell-free nucleosome indicates efficacy of said agent or, optionally, wherein decrease in the level of said cell-free nucleosome indicates efficacy of said agent. In another embodiment, the present invention relates to a method for the selection of a suitable therapeutic agent for treatment of an inflammatory disorder comprising measuring the level of cell-free nucleosomes in a serum or plasma sample isolated from a subject pre and post treatment, wherein a suitable therapeutic agent is selected based on the level of the cell-free nucleosome in the sample. Optionally, decrease in the level of said cell-free nucleosome indicates a suitable therapeutic agent.

The therapeutic agent of the present invention comprises a pharmaceutical composition, a signaling molecule, cells, manipulated cells, or combinations thereof. The pharmaceutical composition includes any immune modulating drug used for treating an inflammatory disorder, in particular, for treating RA, a drug of biologic provenience such as a drug containing antibodies or cell surface bound, intracellular or soluble receptors, an anti-inflammatory drug or combinations thereof. A signaling molecule is, for example, a kinase, or proteases of human or other origin. Optionally, the therapeutic agent is suitable for extracorporeal treatment of the subject such as plasmapheresis or dialysis.

As used herein, the terms "treatment," "treating," and the like, refer to obtaining a desired pharmacologic and/or physiologic effect. The effect may be prophylactic in terms of completely or partially preventing an inflammatory disorder and/or may be therapeutic in terms of a partial or complete cure for an inflammatory disorder and/or adverse effect attributable to said disorder. "Treatment" as used herein also covers any treatment of an inflammatory disorder in a subject, and includes: (a) preventing the disorder from occurring in a subject which may be predisposed to the disorder but has not yet been diagnosed as having it; (b) inhibiting the disorder, i.e., preventing its development; (c) relieving the disorder, i.e., causing regression of the disorder; and (d) ameliorating or alleviating one or more symptom(s) of the disorder.

In another aspect of the invention a method for monitoring an inflammatory disorder activity is provided. Said method comprising measuring the level of cell-free nucleosomes in a serum or plasma sample isolated from a subject, correlating the measured level of cell-free nucleosomes with the activity of the inflammatory disorder, wherein the measured level of cell-free nucleosomes in the sample compared to a predetermined value indicates the extent of activity. For example, the pattern of change in the level of cell-free nucleosome prior and following an intervention indicates the likelihood of a favorable response or lack of a favorable response in a given case.

As used herein, the terms "Measuring" or "measured" and the like refer to determining and/or detecting the presence, absence, quantity, amount, or effective amount of cell-free nucleosomes in a sample, optionally, serum or plasma sample, including the concentration levels of cell-free nucleosomes in said sample. Optionally, measuring the level of cell-free nucleosomes comprises detecting the cell-free nucleosomes according to any of the preceding embodiments. Measuring can be performed by any methods known in the art. However, it is advantageous to measure the level of cell free nucleosomes via detection methods according to the present invention and any of the disclosed embodiments.

In further embodiment, the present invention provides a method for detecting the effect of an environmental stimulus on a subject. The method comprises measuring the level of cell free nucleosomes in a serum or plasma sample, correlating the measured level of cell-free nucleosomes with the effect of the stimulus on the subject, wherein the measured level of cell-free nucleosomes in the sample compared to a pre-determined value indicates the extent of the stimulus effect on the subject.

The environmental stimulus is, for example, is, for example, the inhalation of air of which concentrations and/or pressures of component gases are altered compared to the sea level, of air containing particulate, aerosolized or gaseous elements or molecules not normally present in significant concentrations in clean air, and/or into the upper and/or lower airways, ingestion of solids or particulates of solids, liquids, suspensions of solids in liquids, solutions of solids in liquids or of liquids in liquids, or of said forms of matter on the skin or mucous membranes.

"Predetermined value" according to the present invention can be based upon the level of cell-free nucleosome in comparable samples, optionally serum or plasma samples obtained from the general population or from a selected population. For example, the selected population may be comprised of subjects free of symptoms of an inflammatory disease. The predetermined value can also be based upon the measured level of cell-free nucleosomes from a subject, in particular, a serum or plasma sample isolated from a subject at a certain point of time. The predetermined value can also be determined by obtaining the sample from the subject prior to administration of a therapeutic agent to the subject and measuring the level of cell-free.

In further embodiment, the present invention provides a method for detecting NETosis in a subject, said method comprises detecting cell-free nucleosomes in a serum or plasma sample isolated from said subject, wherein the presence of the cell-free nucleosomes is indicative of NETosis.

In another embodiment, the present invention relates to cell-free nucleosomes for use in detecting, diagnosing, prognosing an inflammatory disorder, in monitoring and/or predicting the efficacy of a therapeutic agent on an inflammatory disorder, in selecting a therapeutic agent for use in treatment of an inflammatory disorder, in detecting NETosis, in monitoring an inflammatory disorder activity, or in detecting the effect of an environmental stimulus on a subject.

In further embodiment of the invention, a composition for use in detecting, diagnosing, and/or prognosing an inflammatory disorder, for use in monitoring and/or predicting the efficacy of a therapeutic agent on an inflammatory disorder, for use in selecting a therapeutic agent for use in treating an inflammatory disorder is provided, for use in detecting the effect of an environmental stimulus on a subject, or for use in detecting NETosis in a subject.

The composition comprises one or more reagents which binds specifically with cell-free nucleosomes in a serum or plasma sample isolated from a subject.

In one embodiment, the one or more reagents of the composition binds specifically with a histone or modified histone protein and with a DNA or modified DNA of the cell-free nucleosome and wherein specific binding of the one or more reagents to the histone or modified histone protein and to DNA or modified DNA of the cell-free nucleosome is indicative of the presence of said inflammatory disorder. Optionally, the one or more reagents binds specifically with a histone protein and modified DNA of the cell-free nucleosome. Optionally, the one or more reagents binds specifically with a modified histone protein and DNA of the cell-free nucleosome.

In another embodiment, the one or more reagents of the composition binds specifically with a modified histone protein and/or modified DNA of the cell-free nucleosome.

In another embodiment, the present invention provides a composition comprising one or more reagents which binds specifically with cell-free nucleosomes in a serum or plasma sample isolated from a subject for use in detecting, diagnosing, and/or making a prognosis of an inflammatory disorder, wherein the reagents comprises a first reagent and a second reagent, wherein one of said first or said second reagent binds specifically with a DNA or a modified DNA of the cell-free nucleosome and the other of said first or second reagent binds specifically with a histone protein or a modified histone protein of the cell-free nucleosome. Optionally, one of said first or said second reagent binds specifically with a DNA of the cell-free nucleosome and the other of said or second reagent binds specifically with a modified histone protein of the cell-free nucleosome. Optionally, one of said first or said second reagent binds specifically with a modified DNA of the cell-free nucleosome and the other of said or second reagent binds specifically with a histone protein of the cell-free nucleosome.

In another embodiment, the present invention provides a method for detecting an inflammatory disorder in a subject comprising administering to a serum or plasma sample isolated from a subject a composition according to any of the preceding embodiments and detecting the binding of said one or more reagents with the cell-free nucleosomes, in particular, with the modified histone protein and/or modified DNA of the cell-free nucleosome or with the histone protein and the modified DNA of the cell-free nuclesome or with the modified histone and DNA of the cell-free nucleosomes or detecting the binding of said first and said second reagent with the cell-free nucleosomes, in particular, detecting binding of one said first or said second reagent with DNA or modified DNA of the cell-free nucleosome and binding of the other of said first or second reagent with a histone protein or a modified histone protein of the cell-free nucleosome, optionally, detecting binding of one of said first or said second reagent with a DNA of the cell-free nucleosome and binding of the other of said or second reagent with a modified histone protein of the cell-free nucleosome, optionally, detecting binding of one of said first or said second reagent with a modified DNA of the cell-free nucleosome and binding of the other of said or second reagent with a histone protein of the cell-free nucleosome, wherein specific binding of said one or more reagents or said first and second reagent is indicative of the presence of said inflammatory disorder. As used herein, "administration" or "administering" refers to any suitable method of providing the composition to the subject.

In another embodiment, the present invention provides kits comprising any of the compositions according to the present invention and any of the disclosed embodiments.

In another embodiment, the present invention relates to use of methods, compositions, kits, and nucleosomes according to the present invention and any of the disclosed embodiments for detecting, diagnosing, and/or prognosing an inflammatory disorder, for detecting NETosis, for predicting and/or monitoring the efficacy of a therapeutic agent for use in treating an inflammatory disorder, for the selection of a therapeutic agent for use in treating an inflammatory disorder, for monitoring an inflammatory disorder activity, and/or for detecting the effect of an environmental stimulus on a subject.

### Materials and Methods:

### Human Subjects

All patients (32 RA patients) fulfilled the American College of Rheumatology classification criteria for RA. Healthy volunteers (41 in number), matched for gender and age, were recruited at the Blood Bank of the Swiss Red Cross, Basel. Inclusion criteria for healthy controls were fair general condition, age ≥ 28 and ≤ 70 years, blood pressure systolic ≤ 140, diastolic ≤ 100. Exclusion criteria were current or previous systemic autoimmune disease, asthma, reconvalescence after major illness, surgery, current medication with corticosteroids, immunosuppressive agents and malignant neoplasia or chemotherapy within 5 years before recruitment for the study. RA patients fulfilled the revised 1987 ACR criteria, had active disease as assessed by a DAS score examination, were from age ≥ 27 to ≤ 70 years and had no other systemic autoimmune disease, including ankylosing spondylitis and psoriatic arthritis. Blood pressure systolic ≤ 140, diastolic ≤ 100. Exclusion criteria were corticosteroids ≥ 40 mg equivalent of prednisone daily and those mentioned above for healthy controls.

### Preparation of plasma and serum

Plasma and serum was collected and processed as known in art *(for example, see* X. Y. Zhong et al., Increased concentrations of antibody-bound circulatory cell-free DNA in rheumatoid arthritis. Clin Chem 53, 1609 (Sep, 2007*)).* Samples were used for experiments immediately or stored at -70°C until further analysis.

### Cell isolation

Human neutrophils were isolated using a Dextran-Ficoll method as used in art *(See for example,* A. K. Gupta, P. Hasler, W. Holzgreve, S. Gebhardt, S. Hahn, Induction of neutrophil extracellular DNA lattices by placental microparticles and IL-8 and their presence in preeclampsia. Hum Immunol 66, 1146 (Nov, 2005*)).* Cell viability after isolation routinely was 96-98%, with a purity of the isolated neutrophils was > 95%. In all experiments, neutrophils at a concentration of 2 x 10⁵/ml were seeded in 24-well tissue-culture plates and allowed to settle for 1 hour, at 37°C with 5% CO₂ prior to stimulation or further analysis as required for a period of up to 3 hours under identical conditions.

### Cell free DNA isolation and quantification

Cell free DNA was extracted from 850 µl plasma or serum using the QIAamp Circulating Nucleic Acid Kit (Qiagen) and quantified by TaqMan^{®} real-time PCR (StepOne™ Plus Real-Time PCR System, Applied Biosystems), using an assay specific for the glyceraldehyde-3-phosphate dehydrogenase (GAPDH)gene locus *(see* X. Y. Zhong et al., Increased concentrations of antibody-bound circulatory cell-free DNA in rheumatoid arthritis. Clin Chem 53, 1609 (Sep, 2007*)).*

Quantification of neutrophil elastase, myeloperoxidase, peptidyl arginine deiminase 4 and cell-free nucleosomes

The concentration of neutrophil elastase (NE), myeloperoxidase (MPO), peptidyl arginine deiminase 4 (PAD4) was assessed by sandwich ELISA using the PMN Elastase/al-PI Complex ELISA Kit (Calbiochem), human MPO ELISA Kit (Hycult Biotech), the human Peptidyl Arginine Deiminase Type IV (PAD4) ELISA Kit (USCN Life Science), respectively. Cell-free nucleosome concentrations were measured using the Human Cell Death Detection ELISA^{PLUS} (Roche Diagnostics). For the analysis of cell-free nucleosomes in cell culture supernatants, incubation with DNaseI (Roche Diagnostics) (10 U / 5 min) was performed as described previously in H. Parker, A. M. Albrett, A. J. Kettle, C. C. Winterbourn, Myeloperoxidase associated with neutrophil extracellular traps is active and mediates bacterial killing in the presence of hydrogen peroxide. J Leukoc Biol 91, 369 (Mar, 2012*).*

### MPO/DNA and histone/MPO complexes quantification

MPO/DNA and histone/MPO complexes were quantified utilizing a modified capture ELISA *(*K. Kessenbrock et al., Netting neutrophils in autoimmune small-vessel vasculitis. Nature medicine 15, 623 (Jun, 2009*)).*

### ROS generation analysis

Cellular ROS generation was measured using a 2', 7'-dichloro dihydro fluorescein diacetate (DCFH-DA) assay *(*C. P. LeBel, H. Ischiropoulos, S. C. Bondy, Evaluation of the probe 2',7'-dichlorofluorescin as an indicator of reactive oxygen species formation and oxidative stress. Chem Res Toxicol 5, 227 (Mar-Apr, 1992*)).* Neutrophils (1 x 10 cells/ml) were incubated for 30 min with 25 µM DCFH-DA. Fluorescence was measured by flow cytometry (FACSCalibur; BD Biosciences) at an excitation wavelength of 485 nm and an emission wavelength of 535 nm.

### Scanning electron microscopy of Neutrophil extracellular traps

Neutrophils were seeded on poly-L-lysine coated coverslips, stimulated with PMA for 60 min, fixed with 2,5% glutaraldehyde (Sigma Aldrich) and dehydrated with a graded ethanol series (30%, 50%, 70%, 100%) *(*A. K. Gupta, P. Hasler, W. Holzgreve, S. Gebhardt, S. Hahn, Induction of neutrophil extracellular DNA lattices by placental microparticles and IL-8 and their presence in preeclampsia. Hum Immunol 66, 1146 (Nov, 2005*)).* Specimens were coated with 2nm platinum and analyzed with a Philips XL-30 ESEM scanning electron microscope (Philips) at the Centre for Microscopy (ZMB), Biozentrum, University of Basel.

Immunohistochemical staining and quantification of Neutrophil extracellular traps 2 x 10⁵/ml isolated neutrophils, were seeded on 13 mm poly-lysine-coated glass coverslips (BD Biosciences) in 24-well tissue-culture plate wells and allowed to settle prior to stimulation as described above. Coverslips were rinsed with ice-cold HBSS and the cells fixed with 4% paraformaldehyde for 15 min at room temperature. Nonspecific binding sites were blocked overnight (HBSS with 10% goat serum, 1% BSA, 0.1% Tween20, and 2 mM EDTA) at 4°C. Neutrophil extracellular traps were detected with primary antibodies to: rabbit anti-neutrophil elastase (Abcam), rabbit anti-myloperoxidase (Dako), rabbit anti-peptidyl arginine deiminase (Abcam), mouse anti-histone, (H1+core proteins, Millipore) and rabbit anti-citrullinated H3 (Abcam). Secondary antibodies were goat anti-rabbit IgG AF555 and goat anti-rabbit IgG AF488 (Invitrogen). DNA was stained with DAPI (4',6-diamidino-2-phenylindole, Sigma) and Neutrophil extracellular traps were visualized by fluorescence and/or confocal microscope as previous.

### Protein isolation and western blotting

Total protein was isolated by NucleoSpin^{®} TriPrep kit (Macherey-Nagel) from 3 x 10⁶ neutrophils. Proteins from the nuclear and cytoplasmic fractions were isolated using the Nuclear and Cytoplasmic Protein Extraction Kit (Thermo Scientific). Western blotting was performed using AnykD™ Mini-PROTEAN® TGX Gels (Biorad) and nylon/nitrocellulose membranes (Biorad). Primary and secondary antibodies utilized were: rabbit anti-PAD4 (Abcam), rabbit anti-MPO (Cell Signaling), mouse anti-β-Actin (Sigma), goat anti-Mouse and/or anti-Rabbit, Human ads-HRP (Southern Biotech). HRP activity was detected by using SuperSignal^{®} West Pico Chemiluminescent Substrate (Thermo Scientific). Equal loading was verified using beta-actin or histone H3, where appropriate. Western blot analysis of citrullinated H3 protein was performed according to Shechter et al. *(*D. Shechter, H. L. Dormann, C. D. Allis, S. B. Hake, Extraction, purification and analysis of histones. Nat Protoc 2, 1445 (2007*)).*

### RNA isolation and quantitative real-time PCR

Total RNA was isolated using RNeasy Mini Kit (Qiagen). TaqMan real-time quantitative RT-PCR was performed using the Applied Biosystems StepOne Plus^{™} Instrument (Applied Biosystems) and TaqMan Gene Expression Assay primer/probe sets (Applied Biosystems) for PAD4 (HS00202612_ml), MPO (HS00924296_ml), Neutrophil elastase (HS00236952_ml) and beta-2 microglobulin (HS99999907_ml). Data were normalized using the housekeeping gene B2M and relative quantification was calculated by 2^{-DDCt} analysis *(*K. J. Livak, T. D. Schmittgen, Analysis of relative gene expression data using real-time quantitative PCR and the 2(-Delta Delta C(T)) Method. Methods 25, 402 (Dec, 2001*)).*

### Statistical analysis

All data are presented as means ± SE. Descriptive statistics for continuous parameters consist of median and range whereas categorical variables are expressed as percentages. Characteristics between the patients and the healthy controls were compared by the Mann-Whitney t test with a Welch post test correction. Statistical significance in multiple comparisons was determined by one-way analysis of variance (ANOVA) with a Dunn's post test correction. A P value < 0.05 was considered statistically significant. Receiver operating characteristic (ROC) curves were calculated and the values for the area under the curve (AUC) with corresponding standard errors of means were estimated. Statistical analysis was performed in GraphPad Prism version 5.0b for MacOSX (GraphPad Software Inc.).

### Results and Advantages:

### Demographics and patient population:

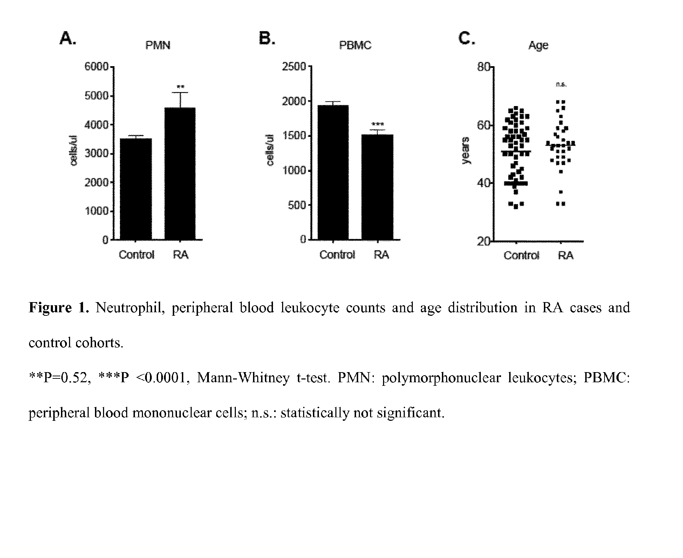

Patient data are provided in Table 1 and Figure 1.

### RA blood samples exhibit increased PMN NETosis during clotting:

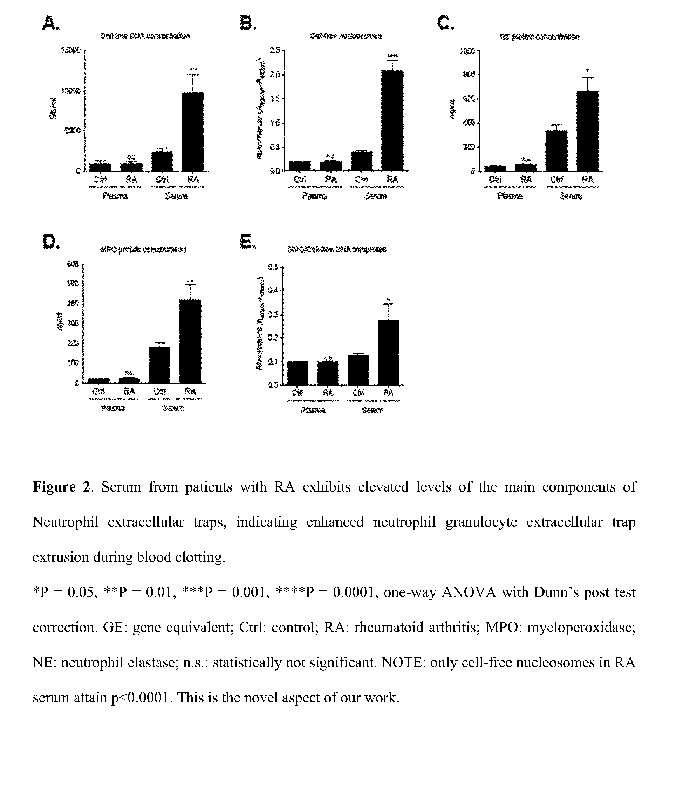

Cell-free DNA concentrations were significantly higher in blood samples permitted to clot from RA cases than in matched healthy control sera (Fig. 2A). An analogous increase in cell-free nucleosomes, neutrophil elastase (NE) and myeloperoxidase (MPO) concentrations was observed in RA serum (Fig. 2B to D). This also held true for cell-free nucleosomes associated with MPO, indicating a NETotic origin of this material (Fig. 2E). As no such elevation was noted in these factors in rapidly processed plasma samples, these data suggest a propensity for RA PMN to undergo increased NETosis during clotting.

In all assays (Fig. 2 A-E) RA-derived serum exhibits elevated concentration levels of these main Neutrophil extracellular traps components compared to healthy donors.

### Clinical application of NETosis associated serum markers:

### 1. Diagnosis of suspected RA:

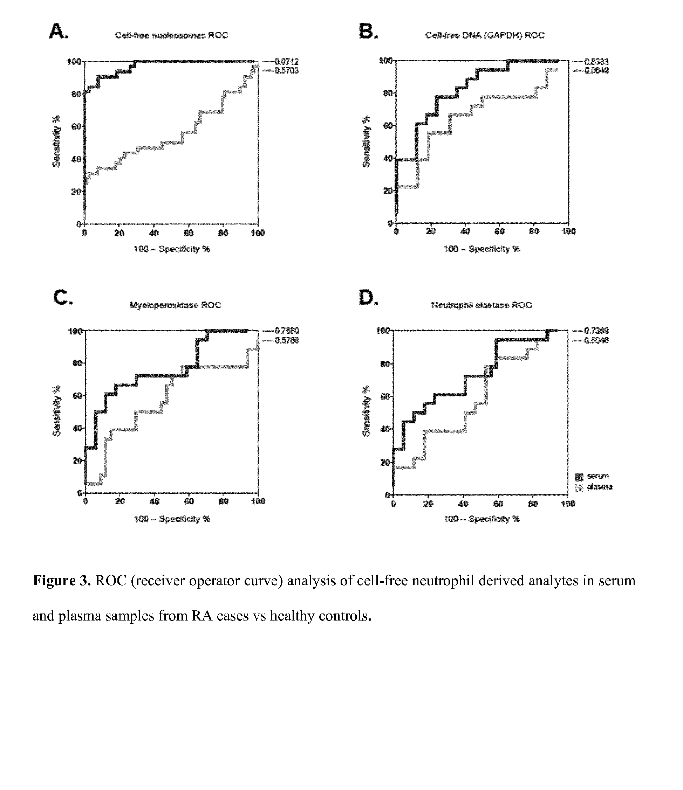

To determine whether the parameters associated with NET production measured in the peripheral blood could be clinically relevant, the data were displayed as ROC curves (Table 2 / Figure 3). This analysis yielded the surprising result that the ROC analysis of cell-free nucleosomes in RA patient serum had an AUC of 0.97 (Table 2; Fig. 3A), significantly higher than for any of the other parameters examined (Fig. 3A-D). This value translates into a sensitivity of 0.91, with a specificity of 0.92, at a cut-off rate of 0.78.

### 2. Monitoring of therapeutic efficacy:

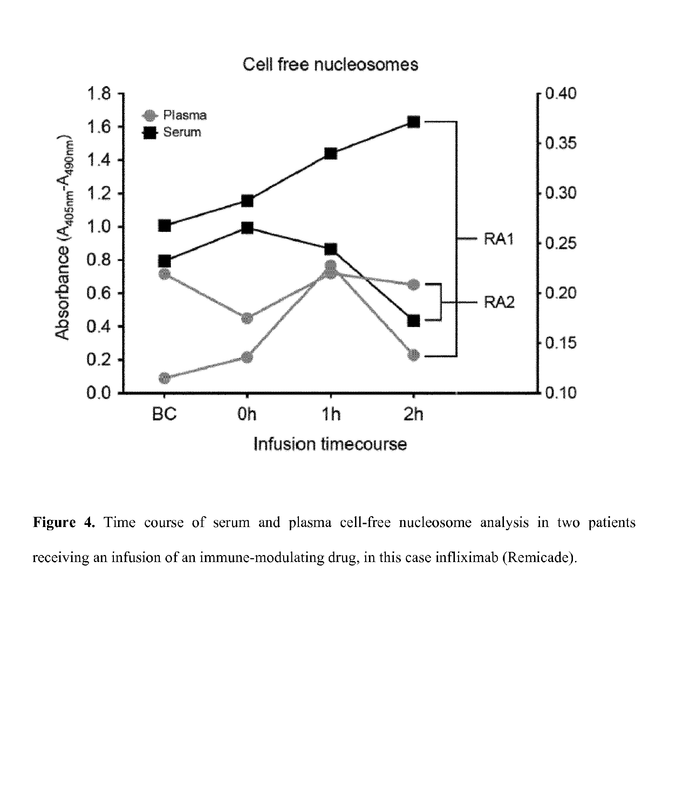

To determine whether this marker would be useful to assess efficacy of therapeutic response, blood samples were taken before infusion of an immune-modulating drug (BC and 0hr) and at set time points post infusion (1 hr and 2 hr). Cell-free nucleosome levels were measured in serum and plasma samples (Figure 4). Figure 4 illustrates a time course of serum and plasma cell-free nucleosome analysis in two patients receiving an infusion of an immune-modulating drug, in this case infliximab (Remicade). Blood samples were taken before infusion (BC), immediately prior to infusion (0hr), immediately post-infusion (1hr) or 1 hour post infusion (2hr). In patient RA1 an increase in serum cell-free nucleosomes is noted, as well as a peak in plasma cell-free nucleosomes at 1 hour. In patient RA2, there is a decrease in serum and in plasma cell-free nucleosomes. This indicates that the biomarker of the present invention, cell-free nucleosome, optionally, in combination with the detection methods of the present invention can be used advantageously to monitor drug response and therapeutic efficacy.

### RA PMN exhibit enhanced spontaneous NET generation in vitro

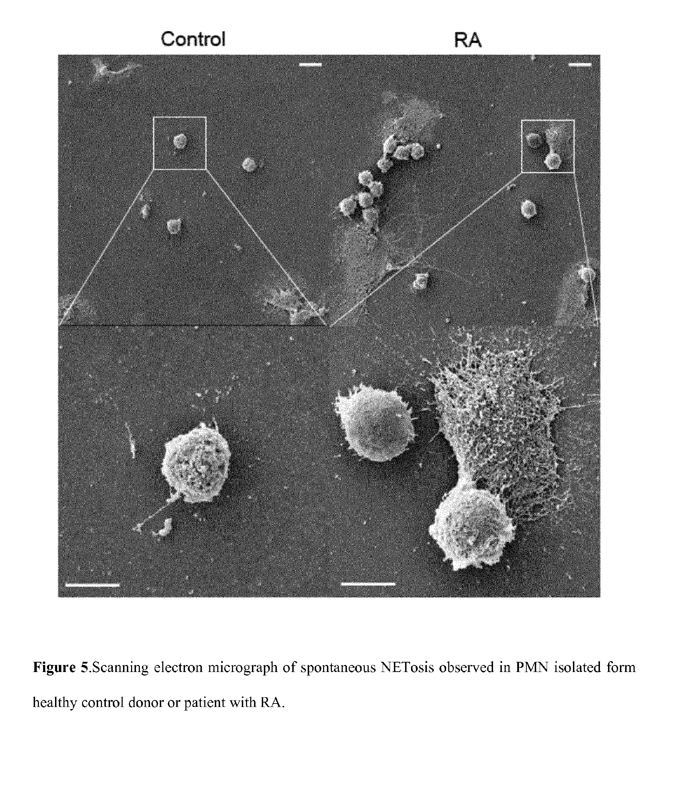

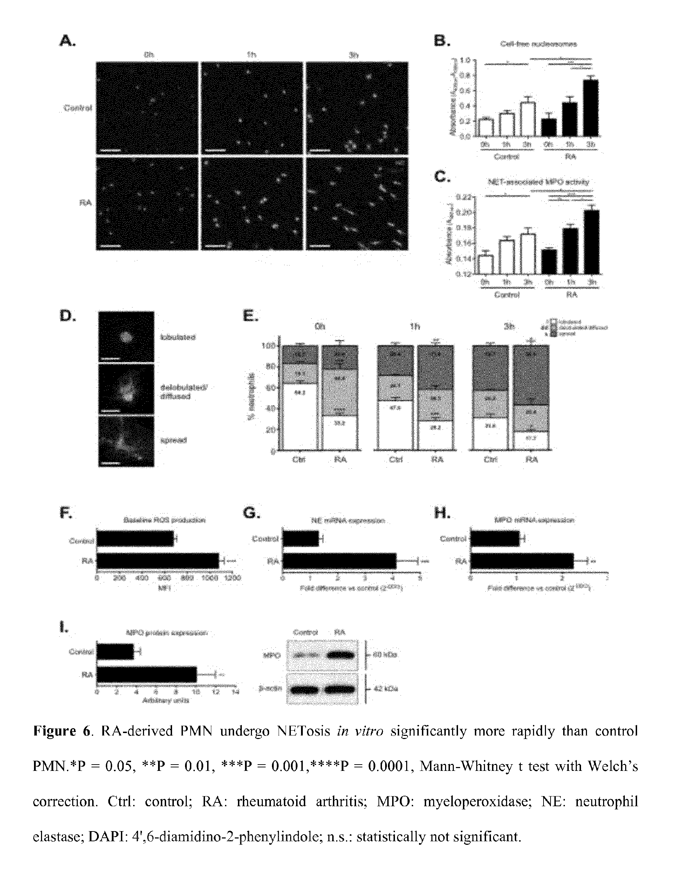

Scanning electron microscopy (Fig. 5), fluorescence microscopy (Fig. 6A) and analysis of cell-free nucleosomes (Fig. 6B), or Neutrophil extracellular traps-associated MPO activity (Fig. 6C), demonstrated that isolated PMNs extrude Neutrophil extracellular traps spontaneously *in vitro.* Compared with the normal controls, Neutrophil extracellular traps were generated more rapidly and to a far greater magnitude by PMN isolated from RA cases than controls, which was most pronounced after 3 hours incubation (Fig. 6A to D). During NET formation, the morphology of the PMN nucleus progresses from the familiar lobulated to a diffused and then to a spread phenotype (Fig. 6D) *(*V. Papayannopoulos, K. D. Metzler, A. Hakkim, A. Zychlinsky, Neutrophil elastase and myeloperoxidase regulate the formation of neutrophil extracellular traps. J Cell Biol 191, 677 (Nov 1, 2010*)).* At baseline, the nuclei of PMNs from healthy controls were predominantly lobulated, while the majority of isolated from RA cases were diffused or spread (Fig. 6E). Spontaneous progression was more pronounced in RA than in normal PMNs, with significantly more spread and less lobulated forms in the RA PMNs after 3 hours (Fig. 6E).

### Expression profile of NETosis signaling components

NETosis has been shown to be dependent on biochemical signaling pathways, among which are the generation of reactive oxygen species (ROS) by NADPH (nicotinamide adenine dinucleotide phosphate) oxidase, the action of NE in combination with MPO, and histone citrullination by PAD4 (peptidyl arginine deiminase 4*) (*T. A. Fuchs et al., Novel cell death program leads to neutrophil extracellular traps. J Cell Biol 176, 231 (Jan 15, 2007*); and* Q. Remijsen et al., Dying for a cause: NETosis, mechanisms behind an antimicrobial cell death modality. Cell Death Differ 18, 581 (Apr, 2011*)).*

RA-derived PMN exhibited increased basal intracellular iROS levels (Fig. 6F), as well as increased levels of NE (Fig. 6G) or MPO (Fig. 6H and I), as determined by real-time PCR or western blotting.

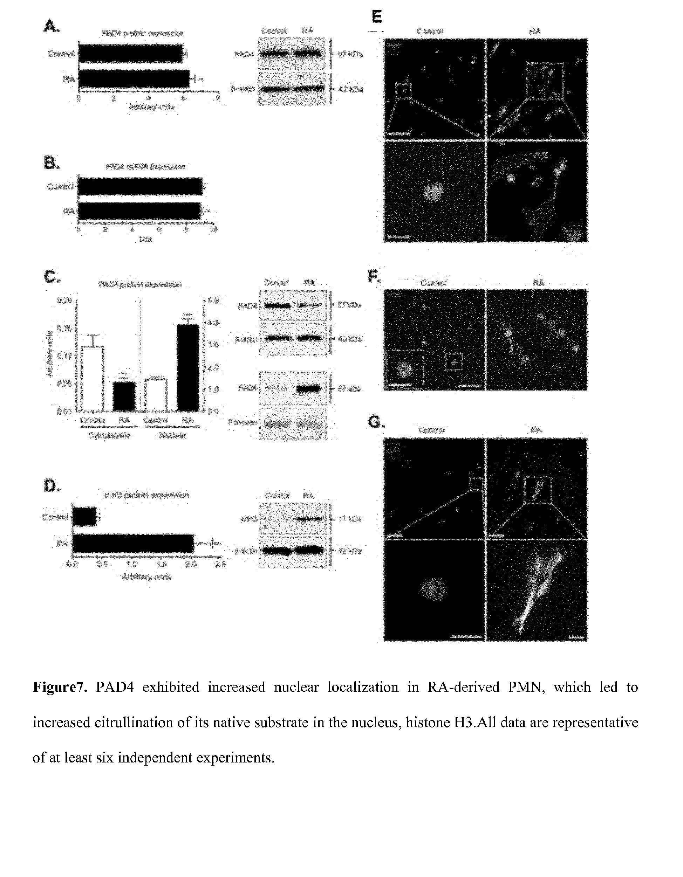

Surprisingly, neither PAD4 mRNA nor total cellular protein expression showed any discernible difference between RA cases and controls (Fig. 7A and B). Since PAD4 is translocated to the nucleus upon activation *(*Y. Wang et al., Histone hypercitrullination mediates chromatin decondensation and neutrophil extracellular trap formation. J Cell Biol 184, 205 (Jan 26, 2009*);* P. Li et al., PAD4 is essential for antibacterial innate immunity mediated by neutrophil extracellular traps. JExp Med 207, 1853 (Aug 30, 2010*)),* where it citrullinates histone proteins, such as histone H3, nuclear and cytoplasmic concentrations was examined. This analysis indicated that PAD4 was preferentially located in the nucleus in RA derived PMN, when compared to control PMN (Fig. 7C). Western blot analysis indicated that citrullinated H3 levels were elevated in PMN from RA cases compared to controls (Fig. 7D), providing evidence that the PAD4 in the nucleus was active. Of interest is that PAD4, as well as PAD2, are extruded together with another nuclear material during NETosis (Fig. 7E to G). According to Fig. 7A, no significant difference was noted in total cellular PAD4 expression between PMN from cases with RA and controls as determined by western blotting. This was also apparent for PAD4 mRNA levels as determined by real-time PCR (Fig. 7B). PAD4 protein levels were determined to be greater in the nuclear fraction of PMN from RA cases when compared to healthy controls. There was also a corresponding decrease in the cytoplasmic PAD4 level in RA-derived PMN (Fig. 7C). PAD4 was determined to be extruded on the Neutrophil extracellular traps of RA-derived PMN (Fig. 7D). PAD2 was also determined to be present on the extracellular Neutrophil extracellular traps of RA-derived PMN (Fig. 7E). Citrullinated histone H3 (citH3) levels were increased in RA-derived PMN, indicating a greater activity of PAD4 in this instance (Fig. 7F).

### RA PMN response to stimulatory signals and influence of RA derived sera.

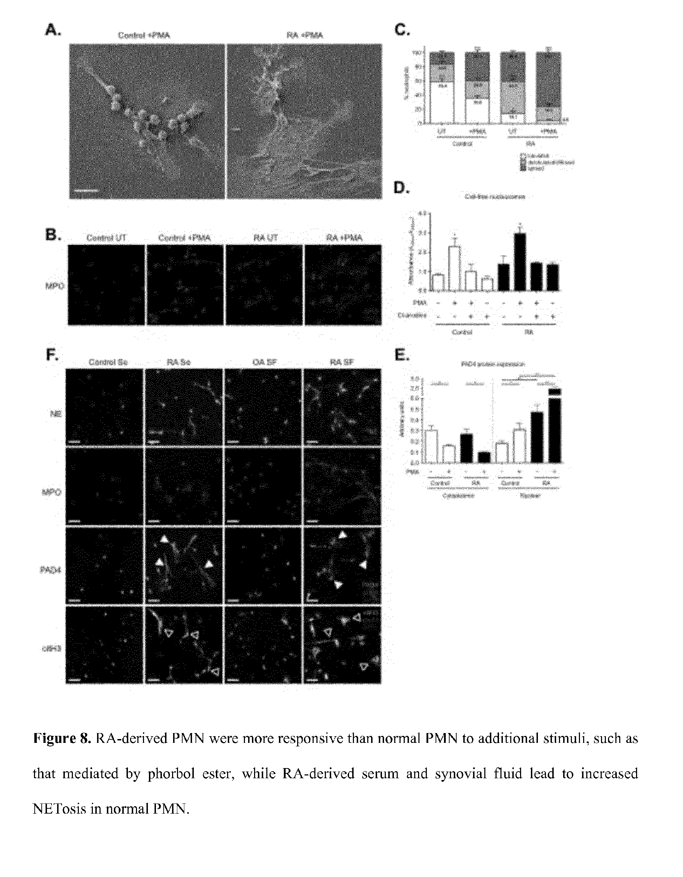

PMNs have been shown to be primed for NETosis in response external activation in certain inflammatory or malignant conditions *(*R. Lande et al., Neutrophils activate plasmacytoid dendritic cells by releasing self-DNA-peptide complexes in systemic lupus erythematosus. Science translational medicine 3, 73ra19 (Mar 9, 2011*);* M. Demers et al., Cancers predispose neutrophils to release extracellular DNA traps that contribute to cancer-associated thrombosis. Proceedings of the National Academy of Sciences of the United States of America 109, 13076 (Aug 7, 2012*)).* Therefore, the response to the phorbol ester PMA (phorbol-12-myristate-13-acetate) was examined, where it was observed that RA-derived PMN responded more vigorously (Fig. 8A and B), progressing more rapidly to a spread nuclear phenotype (Fig. 8C) and producing significantly more Neutrophil extracellular traps within 1 hour, while PMNs from healthy donors responded with moderate NET formation (Fig 5D). Phorbol ester treatment appears to involve activation of PAD4, as it enhanced nuclear localization (Fig. 8E), and its action was reduced by treatment with chloramidine, a PAD4 inhibitor (Fig. 8D). Scanning electron microscopy indicates that RA-derived PMN undergo very vigorous NETosis when stimulated with phorbol ester (PMA) (Fig. 8A). Fig. 8B confirms enhanced NETosis by PMN isolated from RA cases compared to controls when stimulated with PMA by staining for MPO and DAPI. Fig. 8C. Illustrates significant change in nuclear morphology by PMN from RA cases when treated with PMA when compared to control PMN, in that the vast proportion undergo NETosis (spread form) and both the population of the delobulated and lobulated forms is decreased. Figure 8D is an evidence for increased NETosis following treatment with PMA as measured by the presence of cell-free nucleosomes in culture supernatants. Partial inhibition by treatment with the PAD4 inhibitor chloramidine (Cl-amidine). Treatment with phorbol ester (PMA) leads to an increased nuclear presence of PAD4 in PMN isolated from RA cases, and a concomitant decrease in the cytoplasmic fraction (Fig. 8E). According to Fig. 8F. serum (Se) and synovial fluid (SF) from rheumatoid arthritis (RA) but not osteoarthritis (OA) stimulate NETosis in normal PMN. This is evident by immune-fluorescent staining for MPO, PAD4 and citH3.

These data suggest that PMN in RA are primed to undergo increased NETosis following stimulation by a secondary signal.

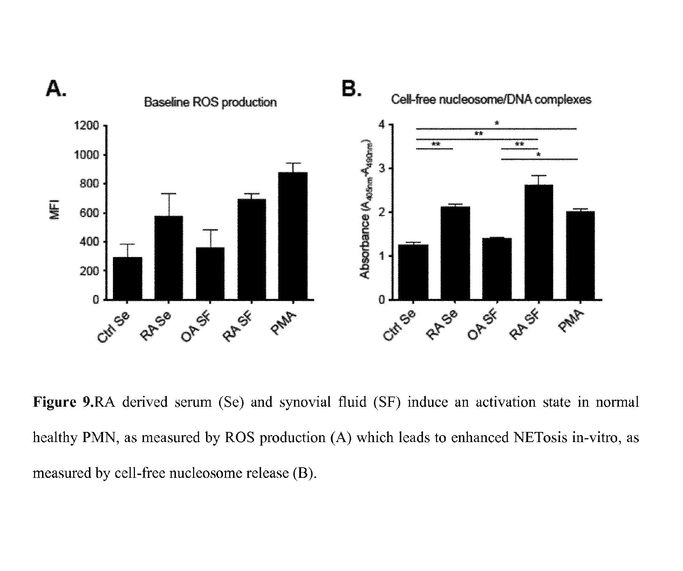

Since it has been described that healthy PMN exhibited increased NETosis upon incubation with SLE patient sera, we examined the effects of RA serum (Se) and synovial fluid (SF). The incubation of normal healthy PMN with RA serum or SF led to an increase in basal ROS production (Fig. 9A), as well as an increase in NETosis (Fig. 8F and Fig. 9B).

### Further results:

Although PMN figure prominently in the joint effusions and inflamed synovial tissue of RA patients *(*R. N. Maini, M. Feldmann, Cytokine therapy in rheumatoid arthritis. Lancet 348, 824 (Sep 21, 1996*)),* the potential roles of NETotic events in the pathophysiology of this disorder have not been addressed to date. This is in contrast to recent observations concerning the involvement of Neutrophil extracellular traps in autoimmune disorders such as SLE, Felty's syndrome, or ANCA-induced SVV (small vessel vasculitis) *(*R. Lande et al., Neutrophils activate plasmacytoid dendritic cells by releasing self-DNA-peptide complexes in systemic lupus erythematosus. Science translational medicine 3, 73ra19 (Mar 9, 2011*);* N. Dwivedi et al., Felty's syndrome autoantibodies bind to deiminated histones and neutrophil extracellular chromatin traps. Arthritis and rheumatism 64, 982 (Apr, 2012*);* G. S. Garcia-Romo et al., Netting neutrophils are major inducers of type I IFN production in pediatric systemic lupus erythematosus. Science translational medicine 3, 73ra20 (Mar 9, 2011*);* K. Kessenbrock et al., Netting neutrophils in autoimmune small-vessel vasculitis. Nature medicine 15, 623 (Jun, 2009*)).* The data of the present invention indicate that in RA patients, circulatory PMNs are primed for NETosis, as was evident from increased intracellular ROS production, enhanced expression of NE and MPO, nuclear translocation of PAD4 and citrullination of histone H3. Furthermore, isolated PMN from RA cases exhibited different nuclear morphometric characteristics, having a lower proportion of the classical lobulated phenotype and *in vitro,* spontaneously progressed to a NETotic phenotype more rapidly than controls.

An intriguing feature of the data of the present invention is the extracellular presence of PAD4 and PAD2 on extruded Neutrophil extracellular traps. Although the citrullination of histone proteins by PAD4 is an important component of the NETotic cascade *(*P. Li et al., PAD4 is essential for antibacterial innate immunity mediated by neutrophil extracellular traps. J Exp Med 207, 1853 (Aug 30, 2010*)),* PAD2 has a broader range of activity (E. Darrah, A. Rosen, J. T. Giles, F. Andrade, Peptidyl arginine deiminase 2, 3 and 4 have distinct specificities against cellular substrates: novel insights into autoantigen selection in rheumatoid arthritis. Annals of the rheumatic diseases 71, 92 (Jan, 2012*)),* and is largely proposed to be responsible for the citrullination of proteins associated with anti-citrullinated peptide antibodies (ACPA) *(*N. Wegner et al., Autoimmunity to specific citrullinated proteins gives the first clues to the etiology of rheumatoid arthritis. Immunol Rev 233, 34 (Jan, 2010*)).* As molecular structures involving the attachment of enzymes to DNA lattices have been shown to increase their catalytic activity enormously, and thereby form the basis of nano-machines or nano-factories *(*F. C. Simmel, DNA-based assembly lines and nanofactories. Current opinion in biotechnology 23, 516 (Aug, 2012*)),* the concomitant extracellular presence of these two enzymes on Neutrophil extracellular traps increases the spectrum of citrullinated proteins and their local concentration significantly. This production of large amounts of immunogenic material is important in the generation of ACPA. Furthermore, the extracellular localization of PAD4 plays a role in the development of anti-PAD4 antibodies observed in cases with RA *(*E. H. Halvorsen et al., Serum IgG antibodies to peptidyl arginine deiminase 4 in rheumatoid arthritis and associations with disease severity. Annals of the Rheumatic Diseases 67, 414 (Mar, 2008*)).* Although PMN in RA cases are primed for NETosis, and exhibit enhanced spontaneous NETosis *in vitro,* they responded very vigorously to additional stimuli, such as that simulated by PMA. This suggests, that *in vivo,* the generation of Neutrophil extracellular traps in cases with RA may be more readily triggered by conventional agonists such as inflammatory cytokines. It will be of interest to examine whether this feature is associated with episodes of RA flares. It is also currently unclear what factor(s) in RA serum or synovial fluid are responsible for inducing this phenotype change in circulatory PMN.

The finding of the present invention that the clotting of blood samples from RA patients triggers the NETotic cascade, as is evident by the increased presence of cell-free nucleosomes has unexpected clinical ramifications, in that this serves to distinguish RA patients from healthy controls with a high degree of specificity. As mentioned previously and to reiterate, although the presence of ACPA has high specificity for the development of RA and have better predictive value than the occurrence of rheumatoid factor (RF), they are only present in approximately two-thirds of RA patients. In addition, ACPA appear to be strongly associated with the "shared epitope" on HLA-DRB1, and may be subject to ethnic differences. Therefore, detection of cell-free nucleosomes is advantageous in detecting also those cases that are ACPA-negative. The association of Neutrophil extracellular traps with RA suggests that targeting PMNs permits more directed diagnostic and therapeutic approaches.

### OTHER EMBODIMENTS

The detailed description set forth above is provided to aid those skilled in the art in practicing the present invention. However, the invention described and claimed herein is not to be limited in scope by the specific embodiments herein disclosed because these embodiments are intended as illustration of several aspects of the invention. The embodiments set forth above can be performed and combined with other disclosed embodiments according to the invention. Any equivalent embodiments are intended to be within the scope of this invention. Indeed, various modifications of the invention in addition to those shown and described herein will become apparent to those skilled in the art from the foregoing description which do not depart from the spirit or scope of the present inventive discovery. Such modifications are also intended to fall within the scope of the appended claims. All publications, patents, patent applications and other references cited in this application are incorporated herein by reference in their entirety for all purposes to the same extent as if each individual publication, patent, patent application or other reference was specifically and individually indicated to be incorporated by reference in its entirety for all purposes. Citation of a reference herein shall not be construed as an admission that such is prior art to the present invention.

**Table 1. Demographics and patient population characteristics versus healthy blood donors.**

| | **Controls** | **RA** | **Statistics** |
|---|---|---|---|
| **Age** | 52.02 ± 1.428 | 54.11 ± 1.434 | P = 0.1622 |
| **Gender** | 36F / 20M | 25F / 8M | - |
| **DAS28** | n.a. | 3.069 ± 1.124 | - |
| **Bone erosion** | n.a. | 23 /10 | - |
| **Serum aCCP Abs** | n.a. | 20 / 13 | - |
| **Serum RF** | n.a. | 19 / 14 | - |
| **Serum ANA** | n.a. | 21 / 12 | - |
| **ESR** | n.a. | 16.82 ± 13.12 | - |
| **CRP** | n.d. | 6.864 ± 5.181 | - |
| **PBMC*** | 1918 ± 68.70 | 1476 ± 68.86 | P < 0.0001 |
| **PMN*** | 3518 ± 126.0 | 4454 ± 451.0 | P = 0.0516 |
| **Therapy** | n.a. | 32 / 1 | - |
| **DMARDS** | n.u. | 28 / 5 | - |
| **Biologics** | n.a. | 31 / 2 | - |

| | | | |
|---|---|---|---|
| n.a.: not applicable;n.u.: not utilized; Abs: antibodies; *: cells/µl; DAS: disease activity score; aCCP: anti-cyclic citrullinated peptide antibodies; RF: rheumatoid factor; ANA: antinuclear antibodies; ESR: erythrocyte sedimentation rate; CRP: C-reactive protein; PBMC: peripheral blood mononuclear cells; PMN: polymorphonuclear leukocytes; DMARDS: disease-modifying antirheumatic drugs. | | | |

**Table 2. AUC values with corresponding standard errors, 95% confidence intervals, P values and standard errors for serum cell free nucleosomes and the 3 different parameters, which were analyzed individually by logistic regression.**

| **Parameter** | **Material** | **AUC** | **95% CI** | **P value** | **S.E.** |
|---|---|---|---|---|---|
| **Cell free nucleosomes** | Serum | 0.9712 | 0.9399 to 1.0030 | < 0.0001 | 0.01598 |
| | Plasma | 0.5703 | 0.4287 to 0.7119 | 0.3078 | 0.07223 |
| **Cell free DNA (GAPDH)** | Serum | 0.8333 | 0.7014 to 0.9652 | 0.0007670 | 0.06729 |
| | Plasma | 0.6649 | 0.4782 to 0.8517 | 0.1013 | 0.09524 |
| **Myeloperoxidase** | Serum | 0.7680 | 0.6097 to 0.9262 | 0.006825 | 0.08071 |
| | Plasma | 0.5768 | 0.3808 to 0.7728 | 0.4380 | 0.09999 |
| **Neutrophil elastase** | Serum | 0.7369 | 0.5720 to 0.9019 | 0.01675 | 0.08414 |
| | Plasma | 0.6046 | 0.4146 to 0.7946 | 0.2909 | 0.09693 |

| | | | | | |
|---|---|---|---|---|---|
| AUC: Area under the curve; 95% CI: 95% confidence interval; S.E.: standard error. | | | | | |

## Claims

1. A method for detecting an inflammatory disorder in a subject, said method comprising detecting cell-free nucleosomes in a serum or plasma sample isolated from said subject, wherein the presence of the cell-free nucleosomes is indicative of the presence of said inflammatory disorder.

2. A method for detecting NETosis in a subject, said method comprising detecting cell-free nucleosomes in a serum or plasma sample isolated from said subject, wherein the presence of the cell-free nucleosomes is indicative of NETosis.

3. A method for predicting or monitoring the efficacy of a therapeutic agent for use in treating an inflammatory disorder, said method comprising measuring the level of cell-free nucleosomes in a serum or plasma sample isolated from a subject pre and post treatment, wherein a change in the level of said cell-free nucleosomes indicates efficacy of said agent.

4. A method for the selection of a therapeutic agent for use in treating an inflammatory disorder, said method comprising measuring the level of cell-free nucleosomes in a serum or plasma sample isolated from a subject pre and post treatment, wherein a therapeutic agent is selected based on the level of the cell-free nucleosome in the sample.

5. A method for monitoring an inflammatory disorder activity or for detecting the effect of an environmental stimulus on a subject, said method comprises measuring the level of cell-free nucleosomes in a serum or plasma sample isolated from a subject, correlating the measured level of cell-free nucleosomes with the activity of the inflammatory disorder or with the effect of an environmental stimulus, wherein the measured level of cell-free nucleosomes in the sample compared to a pre-determined value indicates the extent of inflammatory disorder activity or the effect of the environmental stimulus on the subject.

6. The method according to claim 1 or 2, wherein detecting the cell-free nucleosomes in the serum or plasma sample isolated from said subject comprises contacting the serum or plasma sample with one or more reagents, wherein the one or more reagents binds specifically with a modified histone protein and/or modified DNA of the cell-free nucleosome and wherein specific binding of the one or more reagents to the modified histone protein and/or modified DNA of the cell-free nucleosome is indicative of the presence of said inflammatory disorder.

7. The method according to claim 1 or 2, wherein detecting the cell-free nucleosomes in the serum or plasma sample isolated from said subject comprises contacting the serum or plasma sample with a first reagent and a second reagent, wherein one of said first or said second reagent binds specifically with DNA or modified DNA of the cell-free nucleosome and the other of said first or second reagent binds specifically with a histone protein or a modified histone protein of the cell-free nucleosome and wherein specific binding of the first reagent and the second reagent is indicative of the presence of said inflammatory disorder.

8. A composition comprising one or more reagents which binds specifically with cell-free nucleosomes in a serum or plasma sample isolated from a subject for use in detecting, diagnosing, monitoring and/or making a prognosis of an inflammatory disorder, wherein the one or more reagents binds specifically with modified histone protein and/or modified DNA of the cell-free nucleosomes.

9. A composition comprising a first reagent and a second reagent which bind specifically with cell-free nucleosomes in a serum or plasma sample isolated from a subject for use in detecting, diagnosing, monitoring and/or making a prognosis of an inflammatory disorder, wherein one of said first or said second reagent binds specifically with DNA or modified DNA of the cell-free nucleosomes and the other of said first or second reagent binds specifically with a histone protein or a modified histone protein of the cell-free nucleosomes.

10. A method for detecting an inflammatory disorder in a subject comprising administering to a biological sample isolated from a subject a composition according to claims 8 or 9 and detecting the binding of said one or more reagents or said first and second reagent, wherein specific binding of said one or more reagents or said first and second reagent is indicative of the presence of said inflammatory disorder.

11. A kit comprising the composition according to claims 8 or 9.

12. Use of cell-free nucleosomes in detecting, diagnosing, prognosing an inflammatory disorder, in monitoring and/or predicting the efficacy of a therapeutic agent on an inflammatory disorder, in selecting a therapeutic agent for use in treatment of an inflammatory disorder, in detecting NETosis, in monitoring an inflammatory disorder activity, or in detecting the effect of an environmental stimulus on a subject.

13. Use of the method, composition, and kit according to any of the preceding claims in detecting, diagnosing, prognosing an inflammatory disorder, in monitoring and/or predicting the efficacy of a therapeutic agent on an inflammatory disorder, in selecting a therapeutic agent for use in treatment of an inflammatory disorder, in detecting NETosis, in monitoring an inflammatory disorder activity, or in detecting the effect of an environmental stimulus on a subject.

14. The method according to any of the claims 1-7 or 10, the composition according to claim 8 or 9, the kit according to claim 11, and the use according to claim 12 or 13, wherein said inflammatory disorder is rheumatoid arthritis.

15. The method according to any of the claims 1-7 or 10, the composition according to claim 8 or 9, the kit according to claim 11, and the use according to claim 12 or 13, wherein said inflammatory disorder is preeclampsia, systemic lupus erythematosus, Felty's syndrome, or multiple sclerosis.
